(19) Europäisches Patentamt
European Patent Office
Office européen des brevets

(11) Veröffentlichungsnummer: **0 194 572 B1**

(12) # EUROPÄISCHE PATENTSCHRIFT

(45) Veröffentlichungstag der Patentschrift: **02.01.92**

(21) Anmeldenummer: **86102902.3**

(22) Anmeldetag: **05.03.86**

Die Akte enthält technische Angaben, die nach dem Eingang der Anmeldung eingereicht wurden und die nicht in dieser Patentschrift enthalten sind.

(51) Int. Cl.$^5$: **A61K 31/33**, A61K 31/425, C07D 211/54, C07D 211/84, C07D 213/80, C07D 249/12, C07D 277/32

(54) **Heterocyclische Sulfide, Verfahren zu ihrer Herstellung und pharmazeutische Mittel, die diese Verbindungen enthalten.**

(30) Priorität: **12.03.85 DE 3508665**

(43) Veröffentlichungstag der Anmeldung:
**17.09.86 Patentblatt 86/38**

(45) Bekanntmachung des Hinweises auf die Patenterteilung:
**02.01.92 Patentblatt 92/01**

(84) Benannte Vertragsstaaten:
**AT BE CH DE FR GB IT LI LU NL SE**

(56) Entgegenhaltungen:
**EP-A- 0 093 908**
**AT-B- 238 200**
**AT-B- 367 296**
**DE-A- 2 165 554**
**DE-A- 2 634 409**
**FR-A- 2 368 278**
**GB-A- 1 152 814**
**US-A- 2 886 487**
**US-A- 3 671 643**
**US-A- 3 962 237**
**US-A- 4 018 923**
**EP-A- 0 137 426**
**AT-B- 363 076**
**CH-A- 645 806**
**DE-A- 2 533 605**
**DE-B- 1 667 902**
**FR-M- 601**
**GB-A- 1 604 084**
**US-A- 3 629 473**
**US-A- 3 907 822**
**US-A- 4 002 624**
**US-A- 4 065 584**

(73) Patentinhaber: **HOECHST AKTIENGESELLSCHAFT**
**Postfach 80 03 20**
**W-6230 Frankfurt am Main 80(DE)**

(72) Erfinder: **Scheunemann, Karl-Heinz, Dr.**
**Geisenheimer Strasse 88**
**W-6000 Frankfurt am Main 71(DE)**
Erfinder: **Dürckheimer, Walter, Dr.**
**Im Lerchenfeld 45**
**W-6234 Hattersheim am Main(DE)**
Erfinder: **Blumbach, Jürgen, Dr.**
**Manderscheider Strasse 13b**
**W-6000 Frankfurt am Main 71(DE)**
Erfinder: **Limbert, Michael, Dr.**
**Am alten Birnbaum 21**
**W-6238 Hofheim am Taunus(DE)**
Erfinder: **Schorlemmer, Hans-Ulrich, Dr.**
**Am Kirschenwald 2**
**W-3550 Marburg 21(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

US-A- 4 202 901 US-A- 4 379 159
US-A- 4 447 440

FORTSCHRITTE DER ARZNEIMITTELFORSCHUNG, Band 16, 1972, Basel G.W. CAMIENE, W.J. WECHTER, "Immunosuppression-Agents, Procedures, Speculations and Prognosis" Seiten 67-156

W.O. FOYE, "Principles of Medicinal Chemistry", 2. Auflage, 1981 LEA & FEBIGER, Philadelphia Seiten 91-128, 837-861

Zusatzrecherche, Wien 08.03.1990, Blatt 4 des ERB vom 08.05.1987

Erfinder: **Dickneite, Gerhard, Dr.**
**Zum Neuen Hieb 31**
**W-3550 Marburg(DE)**
Erfinder: **Sedlacek, Hans-Harald, Dr.**
**Sonnenhang 3**
**W-3550 Marburg(DE)**

## Beschreibung

Es ist bekannt, daß die Abwehrmechanismen des lebenden Organismus, die kurz als humorale Immunität und zelluläre Immunität bezeichnet werden, zusammenwirken, um Fremdkörper, die pathogenetische Veränderungen hervorrufen und schädlich sein können, zu neutralisieren und zu eliminieren, vornehmlich Mikroorganismen oder neoplastische Zellen.

Immunologische Untersuchungen ergaben, daß Zusammenhänge zwischen der durch innere oder durch äußere Faktoren provozierten Abnahme der immunologischen Aktivität und der Zunahme der Infektions- oder Tumorkrankheiten bestehen. Daneben entstehen andere Krankheiten durch Veränderungen der Funktionen des Immunsystems. Hierzu gehören beispielsweise Autoimmunkrankheiten oder durch Immunkomplexe hervorgerufene Erkrankungen. Man sucht deshalb seit langem nach Immunstimulantien, d.h. nach Substanzen, die imstande sind, die immunologische Aktivität des Empfängers zu verändern, vorzugsweise zu erhöhen und die aufgrund ihrer hohen Wirksamkeit und guten Verträglichkeit einen breiten Einsatz zur Unterstützung der Abwehrkräfte des Körpers erlauben. Beispiele, die hinsichtlich der Stimulation der Immunität geprüft wurden, sind BCG und C. parvum, ferner die Extrakte des M. tuberculosis und der Brucellen.

Diese Substanzen erzeugen jedoch in den Konzentrationen, wie sie zur Anwendung kommen, deutliche Nebenwirkungen, wie z.B. in verschiedenem Ausmaß lokale Granulome. Die Unkenntnis der genauen Natur der Substanzen erschwert eine systematische Untersuchung mit guter Reproduzierbarkeit der klinischen Ergebnisse. Erwünscht sind somit in diesem Zusammenhang neue Immunstimulantien, die chemisch definierte Substanzen darstellen und geringe Toxizität besitzen, wie z.B. Bestatin, das zur Zeit ein intensiv untersuchtes niedermolekulares Immunstimulans ist und allgemein eine wissenschaftliche Referenzsubstanz darstellt.

Überraschenderweise wurde nun gefunden, daß die erfindungsgemäßen Verbindungen eine hohe immunstimulierende und immunrestaurative Wirkung besitzen, wie sie sich beispielsweise in der DTH-Reaktion auf Schafserythrozyten, in der Aktivierung von mononukleären Phagozyten und in einer ausgeprägten CSF-Aktivität ausdrückt. Diese immunstimulierenden Effekte können beispielsweise auch in einer Erhöhung der Widerstandskraft gegen Infektionen beobachtet werden. Darüberhinaus besitzen die erfindungsgemäßen Verbindungen überraschenderweise zytostatische Wirksamkeit, so beispielsweise gegen das B16-Melanom an der Maus.

Die Erfindung beschreibt somit eine Klasse von immunpharmakologisch und zytostatisch wirksamen Substanzen, die chemisch definiert sind, eine geringe Toxizität besitzen und als solche oder in Kombination mit anderen Wirkstoffen wertvolle Arzneimittel darstellen. Die erfindungsgemäßen Verbindungen weisen einen $LD_{50}$-Wert von mehr als 1000 mg/kg bei intravenöser Injektion bei Mäusen auf. Die wirksame immunmodulatorische und zytostatische Menge liegt bei Wirbeltieren, vorzugsweise warmblütigen Säugern im Bereich von etwa 0,5 bis etwa 100 mg/kg Körpergewicht pro parenteraler oder oraler Gabe, ohne dabei toxische Nebenwirkungen zu zeigen und ist damit für die Behandlung von Krankheiten des Immunsystems sehr gut geeignet.

Die Erfindung betrifft also Sulfide der allgemeinen Formel II

$$Het'\text{-}S\text{-}R^2 \qquad (II)$$

in der Het' für 1,3-Thiazolyl steht, das ein- oder zweifach substituiert ist durch $C_1$-$C_6$-Alkyl;
durch Carboxy-$C_1$-$C_6$-alkyl; durch Carboxy oder durch $C_2$-$C_5$-Acylamino, wobei Acyl für den Rest einer aliphatischen Dicarbonsäure steht,
wobei Het' mindestens eine direkt gebundene Carboxylgruppe oder einen der vorstehend aufgeführten, eine Carboxylgruppe enthaltenen Substituenten trägt und
$R^2$ die Bedeutung besitzt von $C_1$-$C_6$-Alkyl, von $C_1$-$C_6$-Alkyl, das substituiert ist durch Carboxy, $C_1$-$C_4$-Alkoxycarbonyl, Hydroximino, $C_1$-$C_4$-Alkoximino oder Phenyl, das durch Carboxy oder Halogen substituiert sein kann oder von $C_3$-$C_6$-Cycloalkyl, das durch Carboxy substituiert sein kann,
wobei die vorstehend genannten Carboxylgruppen auch in Form ihrer physiologisch verträglichen Salze vorliegen können.

Als $C_1$-$C_6$-Alkylsubstituenten für die Substitution von Het' kommen in Betracht geradkettige oder verzweigte Alkylgruppen mit 1 - 6, vorzugsweise 1 - 4 C-Atomen, wie z.B. Methyl, Ethyl, n- oder i-Propyl, n- oder tert.-Butyl, vorzugsweise Methyl, die gegebenenfalls wieder substituiert sein können durch Carboxy.

Als Beispiele für eine Het' substituierende $C_2$-$C_5$-Acylaminogruppe, in der Acyl für den Rest einer aliphatischen Dicarbonsäure steht, seien genannt 3-Carboxy-propionyl oder 4-Carboxy-butyryl.

Bedeutet $R^2$ einen $C_1$-$C_6$-Alkylrest, so kommt hierfür geradkettiges und verzweigtes Alkyl in Betracht,

wie beispielsweise Methyl, Ethyl, n-Propyl i-Propyl, n-Butyl, n-Pentyl oder n-Hexyl, das gegebenenfalls im Alkylteil ein- oder mehrfach substituiert sein kann durch Carboxy und Alkoxycarbonyl mit 1 - 4 4 C-Atomen, wie Methoxycarbonyl und Ethoxycarbonyl, durch Oximino und Alkoximino mit 1 - 4 C-Atomen, wie insbesondere Methoximino und durch Phenyl, das gegebenenfalls ein- bis dreifach, vorzugsweise einfach substituiert sein kann durch Carboxy und Halogen, wie insbesondere Fluor, Chlor und Brom.

Der Heterocyclus Het' kann ein- oder mehrfach, beispielsweise 1 - 3fach substituiert sein. Bevorzugt sind jedoch solche Verbindungen, die im heterocyclischen Ring einen oder 2 Substituenten tragen. Von diesen Substituenten muß mindestens einer eine direkt an den Heterocyclus gebundene Carboxylgruppe sein oder eine Carboxylgruppe tragen, wie beispielsweise Carboxy-$C_1$-$C_6$-alkyl.

Ist $R^2$ in der oben beschriebenen Weise substituiert, so kann es ein- oder mehrfach, beispielsweise ein- bis dreifach substituiert sein. Bevorzugt ist jedoch eine ein- bis zweifache, insbesondere eine einfache Substitution.

Erfindungsgemäß ganz besonders interessant sind Verbindungen der allgemeinen Formel

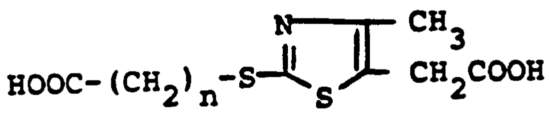

in der n = 1 - 5 sein kann, wobei innerhalb dieser Gruppe die Verbindung, in der n = 3 ist, eine besonders bevorzugte Stellung einnimmt.

Die in den Verbindungen der allgemeinen Formel II auftretenden Carboxylgruppen können auch in Form ihrer physiologisch verträglichen Salze vorliegen, beispielsweise als Alkali- und Erdalkalisalze, wie vorzugsweise die Na, K, Ca, Mg-Salze oder beispielsweise Ammoniumsalze oder substituierte Ammoniumsalze, wie beispielsweise $NH_4^{\oplus}$, Ethanolammonium, Diethanolammonium, Trialkylammonium, wie z.B. Triethylammonium, Tetraalkylammonium, Salze mit basischen Aminosäuren, wie z.B. Lysin, Arginin.

Gegenstand der Erfindung sind weiterhin Verfahren zur Herstellung der erfindungsgemäßen Verbindungen der allgemeinen Formel II. Steht in allgemeiner Formel II $R^2$ und Het' für die oben angegebenen Bedeutungen, so ist das Herstellungsverfahren dadurch gekennzeichnet, daß man eine heterocyclische Thioverbindung der allgemeinen Formel III

HS-Het' (III)

in der Het' die oben angegebene Bedeutung hat, mit einem Alkylierungsmittel der allgemeinen Formel IV,

$R^2$-X (IV)

in der $R^2$ die oben angegebene Bedeutung hat und X für eine leicht abspaltbare Gruppe steht, umsetzt.

Man kann die erfindungsgemäßen Verbindungen der allgemeinen Formel II auch so herstellen, daß man eine heterocyclische Verbindung der allgemeinen Formel V

X-Het' (V)

in der Het' die oben angegebene Bedeutung hat und X für eine leicht abspaltbare Gruppe steht, mit einer Thiolverbindung der allgemeinen Formel VI

$R^2$-SH (VI)

in der $R^2$ die oben angegebene Bedeutung hat, umsetzt.

X in den allgemeinen Formeln IV und V steht für eine leicht austauschbare Gruppe, wie beispielsweise Halogen, bevorzugt Chlor, Brom, Jod, $OSO_2R^3$, wobei $R^3$ für $C_1$-$C_4$-Alkyl, wie bevorzugt Methyl, Halogen-$C_1$-$C_4$-alkyl, wie bevorzugt Trifluormethyl, gegebenenfalls substituiertes Phenyl, wie bevorzugt Phenyl oder p-Tolyl, steht.

Die Umsetzung kann in wässriger Lösung, in einem organischen Lösungsmittel, wie z.B. Alkohol, insbesondere einem niedrigmolekularen Alkohol, beispielsweise Methanol, Ethanol, Propanol oder Isopropanol, in einem Ether, wie z.B. Diethylether, Tetrahydrofuran, Dioxan, in Dimethylformamid, Dimethylacetamid, Aceton, Methylethylketon, Acetonitril, Essigsäureethylester oder einem Gemisch aus den genannten

Lösungsmitteln durchgeführt werden. Es kann zweckmäßig sein, die Reaktion in einem 2-Phasen-System aus Wasser und einem mit Wasser nicht oder nur teilweise mischbaren Lösungsmittel, wie beispielsweise Essigester, Diethylether, Methylenchlorid, Chloroform oder Toluol durchzuführen.

Als bevorzugte Lösungsmittel seien beispielsweise genannt Wasser, Methanol, Ethanol, Aceton, Dimethylformamid, Dimethylacetamid, Tetrahydofuran und Dioxan.

Zur Beschleunigung der genannten Herstellungsmethoden kann es erforderlich sein, einen basischen Stoff in äquimolarer bis etwa zehnfach molarer Menge zuzugeben, wie beispielsweise Natriumhydroxid, Kaliumhydroxid, Natriumcarbonat, Kaliumcarbonat, Natriumbicarbonat oder Kaliumbicarbonat. Auch organische Basen können eingesetzt werden, wie beispielsweise Amine, vorzugsweise Diethylamin, Triethylamin, Diisopropylamin, Ethyldiisopropylamin, N,N-Dimethylanilin, Pyridin, Piperidin, N,N'-Dimethylpiperidin, Morpholin oder N-Methylmorpholin.

Wird die Umsetzung in einem Zweiphasensystem durchgeführt, so kann es vorteilhaft sein, einen Phasentransferkatalysator zuzusetzen, wie beispielsweise quartäre Ammoniumsalze, wie bevorzugt Tetrabutylammoniumchlorid, Tetrabutylammoniumbromid, Tetrabutylammoniumjodid, Tetrabutylammoniumhydrogensulfat, Benzyltrimethylammoniumbromid, Benzyl-dimethyl-n-dodecylammoniumbromid oder tertiäre Phosphoniumverbindungen, wie beispielsweise Ethyltrioctylphosphoniumbromid und Hexadecyltributylphosphoniumbromid.

Die Reaktion kann in einem Temperaturbereich zwischen etwa -20 °C und dem Siedepunkt des verwendeten Lösungsmittels, bzw. Lösungsmittelgemisches, bevorzugt zwischen etwa +5 °C und dem Siedepunkt durchgeführt werden.

Der Wirkstoff kann allein gegeben oder aber auch mit einem oder mehreren, vorzugsweise einem anderen Arzneimittel kombiniert werden, die Infektionen, die beispielsweise durch Bakterien, Pilze oder Viren hervorgerufen werden, und Tumorkrankheiten günstig beeinflussen. Die Wirkstoffe können erfindungsgemäß sowohl parenteral als auch oral verabreicht werden. Für parenterale Verabreichung kommen Lösungen oder Suspensionen des Wirkstoffes in einem pharmazeutisch verträglichen Vektor in Betracht, vorzugsweise Pflanzenöl, wie z.B. Erdnußöl oder Sesamöl, sowie alkoholische Lösungen des Wirkstoffes, z.B. in Ethanol, Propandiol oder Glycerin oder in Gemischen der vorgenannten Lösungsmittel. Zur Herstellung wäßriger Lösungen wird der Wirkstoff vorzugsweise in Form wasserlöslicher, physiologisch verträglicher Salze eingesetzt. Die Zubereitungen können die üblichen Hilfs- und Trägerstoffe enthalten. Als solche kommen beispielsweise Füllstoffe, Emulgatoren, Gleit- und Pufferstoffe und geschmackskorrigierende Agenzien in Frage.

Die Erfindung betrifft daher ebenfalls pharmazeutische Mittel zur Immunstimulation, Immunrestauration und zytostatischen Behandlung, die eine Verbindung der allgemeinen Formel II enthalten.

Im folgenden wird die Einwirkung der Verbindungen auf die Immunantwort der Maus und ihre immunstimulierenden Aktivitäten in verschiedenen in vivo-Standardmethoden beispielhaft erläutert. Die herangezogenen verschiedenen Testmethoden sind bekanntermaßen für die Beurteilung von Immunstimulantien und deren Wirkqualität besonders gut geeignet.

Experiment 1

Wirkung auf die zelluläre immunologische Reaktion vom verzögerten Typ gegen Schafserythrozyten (delayed type hypersensitivity, DTH)

Es wurden Gruppen von 5 weiblichen NMRI-Mäusen mit einem Gewicht von 18 - 20 g intravenös entweder $10^6$ oder $10^9$ rote Blutkörperchen vom Schaf pro Tier verabreicht. Schafserythrozyten gelten in der Immunologie als Standardprüfsubstanz (Antigen) zur Auslösung von zellulären und humoralen Immunreaktionen. Im besonderen gibt dieser Test Auskunft über die Funktionsfähigkeit der T-Zell-abhängigen Komponente (T-Helferzellen) des Immunsystems. Die gemäß Ausführungsbeispiel 7 erhaltene Prüfsubstanz [2-(3-Carboxy-prop-1-yl-thio)-4-methyl-1,3-thiazol-5-yl essigsäure], wurde an den Tagen -3, -2, -1 und 0 in den Konzentrationen 10 - 100 mg/kg in physiologischer Kochsalzlösung intraperitoneal bzw. peroral appliziert. Nach 5 Tagen wurden allen Tieren jeweils 2 x $10^8$ Schafserythrozyten in die Fußsohle injiziert und 24 Stunden später wurde die Schwellung des Fußes gemessen. Die Fußschwellung wird durch eine Hautreaktion vom verzögerten Typ (delayed type hypersensitivity, DTH) ausgelöst und ist, wie dem Fachmann bekannt, ein Maß für die zelluläre Immunantwort (Collins, F.M. und Mackaness, G.B., J. Immunol. 101, 830 - 845, 1968). Die in der Tabelle 1 zusammengestellten Ergebnisse veranschaulichen, daß es durch die Verabfolgung der erfindungsgemäßen Substanz, beispielsweise nach Immunisierung mit $10^6$ oder $10^9$ Schafserythrozyten, zur Steigerung der zellulären Immunantwort kommt. Ein Optimum der Stimulation kann in diesem Experimentalansatz bei der Gabe von 20 - 40 mg/kg Prüfsubstanz beobachtet werden.

Tabelle 1

Immunisierung von Mäusen mit Schafserythrozyten - Wirkung auf die zelluläre Immunantwort (DTH-Reaktion).

| 1 x/Tag i.p. Applikation an Tag -3, -2, -1, 0 von | | % Fußschwellung bei $10^9$ Erythrozyten |
|---|---|---|
| PBS* | | 15,1 ∓ 4,2 |
| Prüfsubstanz | 10 mg/kg | 23,5 ∓ 2,8 |
| " | 20 mg/kg | 27,8 ∓ 3,7 |
| " | 40 mg/kg | 26,3 ∓ 2,5 |
| " | 60 mg/kg | 23,8 ∓ 5,7 |
| " | 80 mg/kg | 20,8 ∓ 3,5 |
| " | 100 mg/kg | 18,2 ∓ 2,7 |
| **1 x/Tag p.o. Applikation an Tag -3, -2, -1, 0 von** | | **$10^6$ Erythrozyten** |
| PBS | | 17,9 ∓ 3,2 |
| Prüfsubstanz | 10 mg/kg | 24,4 ∓ 6,2 |
| " | 20 mg/kg | 27,9 ∓ 8,8 |
| " | 40 mg/kg | 43,5 ∓ 10,5 |
| " | 60 mg/kg | 26,8 ∓ 4,4 |
| " | 80 mg/kg | 25,3 ∓ 3,5 |
| " | 100 mg/kg | 23,2 ∓ 6,5 |

* PBS = Phosphatgepufferte Kochsalzlösung (NaCl: 8000 mg/l; KCl: 200 mg/l, $Na_2HPO_4 \cdot 2H_2O$: 1440 mg/l, $KH_2PO_4$: 200 mg/l)

Experiment 2

Einfluß auf die Stimulation der unspezifischen Immunität - Aktivierung von mononukleären Phagozyten

Hier wurde der Einfluß der nach Ausführungsbeispiel 7 erhaltenen Prüfsubstanz auf die Stimulation von Peritonealmakrophagen bei 6 - 8 Wochen alten NMRI-Mäusen untersucht.

Mäuseweibchen erhielten auf parenteralem oder oralem Wege die Prüfsubstanz in einer Dosis von 25 mg/kg, 50 mg/kg, 100 mg/kg und 200 mg/kg. Der Kontrollgruppe wurde gepufferte Kochsalzlösung verabreicht. Drei Tage nach den Injektionen wurden die Mäuse getötet, und es wurden die Peritonealmakrophagen der Tiere auf ihren Aktivierungszustand hin untersucht. Als Maß der Makrophagenaktivierung wurde zum einen die Sekretion der lysosomalen Enzyme (β-Galactosidase, β-Glucuronidase, N-Acetyl-β-D-glucosaminidase) bestimmt. Auf der anderen Seite konnte in vergleichbaren Makrophagenkulturen die Pinozytose durch die Aufnahme von kolloidalem Gold ($^{198}Au$) - wie sie dem Fachmann bekannt ist - untersucht werden. Die Höhe des oxidativen Stoffwechsels bei Makrophagen gilt als ein weiteres Maß für ihren Aktivierungszustand. Gemessen wird diese Aktivität unter Zuhilfenahme des Biolumaten durch

Bestimmung der Chemolumineszenz.

Zu diesem Zweck wurden entweder in Petrischalen von 30 mm Durchmesser 3 x $10^6$ Makrophagen mit 1 ml TC 199-Kulturmedium oder aber $10^6$ Makrophagen mit 100 µl Medium in Rundboden-Polyäthylen-Röhrchen (zur Bestimmung der Chemolumineszenz) bei 5 % $CO_2$ und 37° C kultiviert.

Nach einstündiger Bebrütung wurden die Kulturen gewaschen, um schwimmende Zellen zu entfernen. Die Chemolumineszenz (Röhrchenkultur) wurde dann direkt bestimmt, während die Petrischalen erneut 24 Stunden bei 37° C bebrütet wurden und danach die Enzym- und Pinozytoseaktivität in den Kulturen bestimmt wurde. Es wurden die nachstehenden Ergebnisse erzielt.

**Tabelle 2**

**Wirkung auf den oxidativen Stoffwechsel bei Maus-Peritonealmakrophagen (Chemolumineszenz in RLE*/15 Minuten).**

| 1 x Applikation | | i.p. | p.o. |
|---|---|---|---|
| PBS | | 3,09 ∓ 0,12 x $10^5$ | 1,86 ∓ 0,09 x $10^5$ |
| | 25 mg/kg | 34,17 ∓ 0,16 x $10^5$ | 31,28 ∓ 0,18 x $10^5$ |
| Prüf- | 50 mg/kg | 86,42 ∓ 0,68 x $10^5$ | 47,24 ∓ 0,36 x $10^5$ |
| sub- | 100 mg/kg | 143,37 ∓ 0,82 x $10^5$ | 80,41 ∓ 0,72 x $10^5$ |
| stanz | 200 mg/kg | 162,04 ∓ 1,19 x $10^5$ | 115,52 ∓ 1,44 x $10^5$ |

* RLE = Relative Lichteinheiten

Die parenterale sowie die orale Behandlung von NMRI-Mäusen mit der gemäß Beispiel 7 hergestellten Prüfsubstanz stimuliert die Makrophagenaktivität und hat damit eine immunitätsstimulierende Wirkung. So wird der oxidative Stoffwechsel bei Makrophagen mit der Generierung von Sauerstoffradikalen und dem damit verbundenen meßbaren Licht deutlich erhöht. Bei Dosierungen von 25 mg/kg aufwärts kommt es zu einer dosisabhängigen Steigerung der Makrophagenaktivität sowohl bei parenteraler als auch oraler Applikation.

Aus der Tabelle 3 ist zu entnehmen, daß Makrophagen von Kontrollmäusen nur geringe Mengen an lysosomalen Enzymen (β-Glucuronidase, β-Galactosidase, N-Acetyl-β-D-glucosaminidase) in den Kulturüberstand abgeben. Mononukleare Phagozyten von Mäusen, die parenteral oder oral mit der Prüfsubstanz 72 Stunden behandelt wurden, sezernieren die o.a. sauren Hydrolasen (β-Glu, β-Gal, N-Ac-Glu) deutlich mehr und weisen damit eine Dosiswirkungskurve auf, die bei allen gemessenen Enzymen eine Überlegenheit gegenüber den Kontrollen erkennen läßt. Es ist ersichtlich, daß die Prüfsubstanz eine stimulierende Wirkung auf die Makrophagenaktivität besitzt und zur Erhöhung der Enzymfreisetzung beiträgt.

Tabelle 3

**Einfluß der Prüfsubstanz auf die Enzymfreisetzung lysosomaler Hydrolasen von Maus-Peritonealmakrophagen.**

| 1 x i.p./p.o. Applikation | | N-Ac-Glu % Freisetzung | ß-Glu % Freisetzung | ß-Gal % Freisetzung |
|---|---|---|---|---|
| PBS | | 7,5/ 6,8 | 13,5/16,4 | 14,9/12,6 |
| Prüf- | 25 mg/kg | 17,2/15,4 | 20,0/17,5 | 24,1/20,4 |
| sub- | 50 mg/kg | 26,0/22,8 | 30,9/25,1 | 33,5/26,9 |
| stanz | 100 mg/kg | 40,8/37,3 | 42,8/38,2 | 52,9/43,6 |
| | 200 mg/kg | 51,5/45,2 | 58,2/49,8 | 65,4/54,8 |

Die quantitative Bestimmung der Pinozytoseaktivität bei mononuklearen Phagozyten wurde nach der Methode von Davies et al. durchgeführt (Davies, P., Allison, A.C. und Haswell, A.D.; Biochem. Biophys. Res. Com. 52, 627, 1973). Dazu wurde readioaktives, kolloidales Gold ($^{198}Au$) mit einer Partikelgröße von 20 nm und einer spezifischen Aktivität von 4 - 12 mCi/mg Au benutzt. Die Ergebnisse in Tabelle 4 veranschaulichen den Effekt der nach Beispiel 7 erhaltenen Prüfsubstanz auf die Endozytoseleistung. Die Pinozytose von kolloidalem Gold ($^{198}Au$) durch Mausperitonealmakrophagen von mit der erfindungsgemäßen Verbindung behandelten Tieren ist im Vergleich mit den Makrophagen von unbehandelten Tieren signifikant und dosisabhängig erhöht.

Tabelle 4

**Der Effekt der Prüfsubstanz auf die Pinozytoseleistung von Mausmakrophagen.**

| 1 x Applikation von | | intraperitoneal | oral |
|---|---|---|---|
| PBS | | 0,201 x $10^3$ cpm | 0,150 x $10^3$ cpm |
| | 25 mg/kg | 0,415 x $10^3$ cpm | 0,365 x $10^3$ cpm |
| Prüf- | 50 mg/kg | 0,558 x $10^3$ cpm | 0,448 x $10^3$ cpm |
| substanz | 100 mg/kg | 0,718 x $10^3$ cpm | 0,661 x $10^3$ cpm |
| | 200 mg/kg | 0,862 x $10^3$ cpm | 0,739 x $10^3$ cpm |

Experiment 3

Erhöhung der Widerstandskraft von Balb/c-Mäusen gegen eine Candida albicans-Infektion

a) Therapeutische Behandlung

Bei der therapeutischen Behandlung einer chronischen Candida albicans Infektion wurden weibliche Balb/c Mäuse (16/Gruppe) intravenös mit Candida albicans (2,5 x $10^5$ cfu/Maus) infiziert (Tag 0). Nach erfolger Infektion wurden die Tiere an 13 aufeinanderfolgenden Tagen (Tage 3 - 15) mit der nach Beispiel 7 erhaltenen Prüfsubstanz intraperitoneal behandelt; die Konzentrationen waren 0,5, 2,0, 5,0, 10,0, 30,0, 60,0

mg/kg. Den Kontrolltieren wurde physiologische Kochsalzlösung verabreicht. An den Tagen 19 und 24 wurde den Tieren Urin entnommen und die Keimzahl bestimmt. Am Tag 26 wurden die Tiere getötet und die Keimzahl und Nekrosen in den Nieren bestimmt.

Die Tabelle 5 zeigt, daß bei allen Konzentrationen der Prüfsubstanz die Urin- und Nierenkeimzahl sowie die Nekrosenbildung in den Nieren vermindert waren. Die optimale Wirkung wurde bei 0,5 - 5,0 mg/kg beobachtet. Somit kann durch therapeutische Gabe der Substanz eine deutliche Verbesserung des Verlaufes einer chronischen Infektion erzielt werden.

## Tabelle 5

Therapie einer chronischen Candida albicans Infektion

| Gruppe | | mit C.albicans infizierte Nieren (%) | nekrotische Nieren (%) | mit C.albicans infizierter Urin d19 (%) | mit C.albicans infizierter Urin d24 (%) |
|---|---|---|---|---|---|
| Kontrolle | | 66 | 75 | 38 | 53 |
| Prüf- | 0,5 mg/kg | 27 | 27 | 0 | 6 |
| sub- | 2,0 mg/kg | 13 | 7 | 6 | 6 |
| stanz | 5,0 mg/kg | 27 | 47 | 0 | 7 |
| | 10,0 mg/kg | 29 | 43 | 7 | 21 |
| | 60,0 mg/kg | 38 | 31 | 13 | 13 |

Tage 3 - 15
10 x i.p.

b) Kombinationstherapie mit einem Antimykotikum

In diesem Experiment wurde die nach Beispiel 7 erhaltene Prüfsubstanz zusammen mit dem Antimykotikum Ketokonazol (Nizoral(R), Janssen) eingesetzt. Weibliche Balb/c-Mäuse (15/Gruppe) wurden intravenös mit Candida albicans ($10^6$ cfu/Maus) infiziert (Tag 0). Drei Gruppen wurden gebildet: Gruppe 1 wurde mit physiologischer Kochsalzlösung behandelt, Gruppe 2 erhielt 70 mg/kg Ketokonazol peroral an 7 aufeinanderfolgenden Tagen (Tage -1-6). Gruppe 3 erhielt zusätzlich zu Ketokonazol 10 intraperitoneale Injektionen von 2 mg/kg der Prüfsubstanz an den Tagen 3 - 30 (jeden 3. Tag). Die Anzahl der gestorbenen Tiere wurde täglich protokolliert und der Zeitpunkt, an dem 50 % der Tiere pro Gruppe gestorben waren ($T_{50}$), ermittelt. Die Tabelle 6 zeigt, daß die Tiere, die eine Kombination von Ketokonazol und der Prüfsubstanz erhielten, eine deutlich höhere Überlebenszeit ($T_{50}$) hatten als die Tiere die Ketokonazol allein erhielten.

Tabelle 6

Kombination der Prüfsubstanz mit einem Antimykotikum. $T_{50}$-Werte in Tagen

| Gruppe | $T_{50}$ |
|---|---|
| 1. Kontrolle | 8 |
| 2. Ketokonazol 70 mg/kg (d-1-6 p.o.) | 18 |
| 3. Ketokonazol 70 mg/kg (d-1-6 p.o.) + Prüfsubstanz 2 mg/kg (10 x i.p., d3-30, jeden 3. Tag) | 30 |

Experiment 4

Stimulation der DTH-Reaktion durch die erfindungsgemäßen Verbindungen

Wie in Experiment 1 beschrieben, wurden NMRI-Mäuse mit den erfindungsgemäßen Substanzen behandelt.

Als Test zur Erfassung der Immunstimulation wurde die DTH-Reaktion überprüft.

Tabelle 7 zeigt die relative Wirksamkeit der Prüfsubstanzen bezogen auf die Verbindung aus Beispiel 7, deren maximale Aktivierung 100 % entspricht (Differenz zwischen Kontrolle und Stimulation). Der Tabelle ist zu entnehmen, daß die DTH-Reaktion bei den mit den Prüfsubstanzen vorbehandelten Tieren deutlich stärker ausgeprägt ist, als bei den entsprechenden Kontrolltieren.

*Tabelle 7*

| Verbindung aus Beispiel Nr. | Dosis (mg/kg) | Applikation | DTH-Reaktion (SRBC) |
|---|---|---|---|
| 7 | 200 | 1 x i.p.Tag 0 | 100 % |
| 39 | 100 | " | 210 % |

Experiment 5

Stimulation der Makrophagenaktivität durch die erfindungsgemäßen Verbindungen

Wie in Experiment 2 beschrieben, wurden NMRI-Mäuse mit den erfindungsgemäßen Verbindungen behandelt.

Als Test zur Erfassung der Immunstimulation wurde die Funktion der Makrophagen (Chemolumineszenz und Enzymaktivität) überprüft.

Tabelle 8 zeigt die relative Wirksamkeit der Prüfsubstanzen bezogen auf die Verbindung aus Beispiel 7, deren maximale Aktivierung (Differenz zwischen Kontrolle und Stimulation) 100 % entspricht. Der Tabelle ist

zu entnehmen, daß im Vergleich zu Makrophagen aus unbehandelten Tieren, diese Zellen auch durch alle Prüfsubstanzen in ihrer Chemolumineszenzreaktion stark stimuliert und in ihrem Gehalt an lysosomalen Enzymen deutlich erhöht waren.

Tabelle 8

| Verbindung aus Beispiel Nr. (100 mg/kg i.p.) | Applikation | Makrophagenaktivität Chemolumineszenz | Exozytose |
|---|---|---|---|
| 1 | 1x i.p. Tag 0 | 29 % | 124 % |
| 7 | " | 100 % | 100 % |
| 15 | " | 49 % | 172 % |
| 39 | " | 35 % | 148 % |

Experiment 6

Einfluß auf die Metastasierung des B16-Melanoms

Zur Behandlung von Metastasen des B16-Melanoms wurde bei weiblichen C57Bl/6 Mäusen (10 Tiere/Gruppe) mit 2 x $10^5$ lebenden B16-Melanomzellen ein Primärtumor induziert. Nach Amputation dieses Tumors metastasiert das B16-Melanom in die Lunge und die Tiere sterben. Die Tiere wurden nach Tumorinduktion entweder an den Tagen 3, 5, 7, 9, 11 und 13 vor oder nach erfolgter Amputation mit 50 mg/kg der nach Beispiel 7 erhaltenen Prüfsubstanz intraperitoneal behandelt. Die Zahl der makroskopisch sichtbaren Metastasen in der Lunge wurde an den Tagen 14, 17, 21, 25 und 28 nach erfolgter Amputation des Primärtumors bestimmt.

Wie aus der Tabelle 9 ersichtlich, war die Zahl der Lungenmetastasen des B16-Melanoms in den behandelten Tiergruppen deutlich geringer als in den entsprechenden Kontrolltieren.

Tabelle 9

Zahl der Lungenmetastasen im Modell des B16-Melanoms

| Tage zur Zählung der Metastasen | Kontrolle $2x10^5$ Tumorzellen Zahl der Metastasen (Todesrate) | Prüfsubstanz (50 mg/kg, 6 x i.p.) Tage 3,5,7,9,11,13 vor Amputation Zahl der Metastasen | Tage 3,5,7,9,11,13 nach Amputation Zahl der Metastasen |
|---|---|---|---|
| 14 | 13 ∓ 5 | 2 ∓ 2 | 6 ∓ 3 |
| 17 | 16 ∓ 3 | 5 ∓ 1 | 6 ∓ 4 |
| 21 | 22 ∓ 2 | 2 ∓ 2 | 5 ∓ 3 |
| 25 | 26 ∓ 2 (5/10) | 12 ∓ 5 | 6 ∓ 5 |
| 28 | 50 (9/10) | 15 ∓ 6 | 12 ∓ 3 |

Experiment 7

Einfluß auf die Knochenmarkskoloniebildung

Hier wurde der Einfluß der nach Beispiel 7 erhaltenen Prüfsubstanz auf die Stimulation von Knochenmarkskolonien bei 6 - 8 Wochen alten B6D2F1 Mäusen untersucht. Mäuseweibchen erhielten die Prüfsubstanz intraperitoneal in den Dosen 2,5 und 5 mg/kg. Einen Tage später wurden die Tiere getötet, die Knochenmarkszellen isoliert und nach den allgemein bekannten Methoden (Metcalf, Immunology 21, 427, 1971 und Stanley et al. J. Exp. Med. 143, 631, 1976) kultiviert. Zur Entwicklung der Knochenmarkskolonien wurde als "CSF"-Quelle (Colony stimulating factor) wie üblich L-Zell-Überstand (15 %) benutzt. Die Kolonien wurden 8 Tage nach der Aussaat gezählt. Wie aus der Tabelle 10 zu ersehen ist, führt die einmalige Gabe von 2,5 oder 5 mg der Prüfsubstanz zu einer deutlichen Steigerung der Koloniebildung bei Knochenmarkszellen sowohl mit als auch ohne Zugabe von CSF (Colony stimulating factor) in vitro.

Tabelle 10

In vivo-Effekt auf die Knochenmarkskoloniebildung

| Prüfsubstanz 1 x i.p. (mg/kg) | Zahl der Knochenmarkskolonien (Tag 8) mit CSF (15 %) | ohne CSF (15 %) |
|---|---|---|
| PBS | 77 ∓ 6 | 2 ∓ 1 |
| 2,5 | 134 ∓ 10 | 56 ∓ 4 |
| 5,0 | 217 ∓ 7 | 117 ∓ 11 |

Die folgenden Beispiele erläutern die Erfindung, ohne sie jedoch einzuschränken.

Beispiel 1:

Stufe 1

2-Mercapto-4-methyl-1,3-thiazol-5-yl-essigsäuremethyl ester

27,0 g (0,14 Mol) 2-Mercapto-4-methyl-1,3-thiazol-5-yl-essigsäure werden in 230 ml Methanol gelöst und 6 ml konz. HCl zugetropft. Bei Raumtemperatur wird 4 Std. nachgerührt, vom Niederschlag abgesaugt, mit wenig Methanol nachgewaschen.
Ausbeute 14,3 g = 49,3 %
Aus der Mutterlauge werden noch 12,0 g = 41,4 % erhalten.
Fp. 134 - 135° C
NMR ($d_6$-DMSO) $\delta$ = 8,67 ppm s breit, 1H, -SH
3,30 ppm s 2H $CH_2$-$COOCH_3$
2,03 ppm s 3H 4-$CH_3$

Stufe 2

2-Methylmercapto-4-methyl-1,3-thiazol-5-yl-essigsäuremethylester

28,0 g (0,14 Mol) Stufe 1 werden in
500 ml Aceton gelöst und mit
38,1 g (0,28 Mol) fein gemahlenem Kaliumcarbonat versetzt. Nach 10 Minuten gibt man
21,6 g =9,5 ml (0,15 Mol) Methyljodid hinzu. Die Temperatur steigt leicht an. Es wird 3 Stunden bei Raumtemperatur nachgerührt. Vom festen Kaliumjodid wird abgesaugt und im Rotationsverdampfer eingeengt. Rückstand 36,1 g gelbes Öl = 100 % d. Th.
NMR ($d_6$-DMSO)$\delta$ 3,50 ppm, s, 2H, -$CH_2$-$COOCH_3$
3,30 ppm, s, 3H, $\overline{CH_2}$-COO$\underline{CH_3}$
2,63 ppm, s, 3H, -S-$CH_3$
2,23 ppm, s, 3H, 4-$CH_3$

Stufe 3

2-Methylmercapto-4-methyl-1,3-thiazol-5-yl-essigsäure

36,1 g (0,166 Mol) Verbindung aus Stufe 2 werden in
50 ml Methanol gelöst und
100 ml 2n-Natronlauge zugefügt. Am Rückfluss wird eine Stunde gekocht, von Methanol abdestilliert und nach Erkalten 2x mit Essigester extrahiert. Mit konz. Salzsäure wird auf pH 3 angesäuert, der Niederschlag abgesaugt und aus Isopropanol umkristallisiert.
Ausbeute 21,2 g = 62,9 % d. Th.
Fp. 159 - 160° C
IR. KBr (1870, 1710, 1550 $cm^{-1}$)
NMR ($d_6$-DMSO)$\delta$ = 3,73 ppm, s, 2H, $\underline{CH_2}$-COOH
2,63 ppm, s, 3H, $\overline{S\text{-}CH_3}$
2,20 ppm, s, 3H, 4-$CH_3$
12,77 ppm, s, 1H, COOH

Beispiel 2:

Stufe 1

2-Aethylmercapto-4-methyl-1,3-thiazol-5-yl-essigsäuremethylester

2,03 g (0,01 Mol) Verbindung aus Beispiel 1, Stufe 1 werden analog Beispiel 1, Stufe 2, mit Kaliumcarbonat und Aethyljodid umgesetzt und aufgearbeitet.
Ausbeute 2,8 g Öl

NMR ($d_6$-DMSO)δ = 3,83 ppm, s, 2H, $CH_2$-$CO_2CH_3$
3,63 ppm, s, 3H, COO-$CH_3$
3,10 ppm, q, 2H, $SCH_2CH_3$
2,23 ppm, s, 3H, 4$CH_3$
1,33 ppm, t, 3H, S-$CH_2CH_3$

Stufe 2

2-Aethylmercapto-4-methyl-1,3-thiazol-5-yl-essigsäure

2,8 g Verbindung aus Stufe 1 werden analog Beispiel 1, Stufe 3, umgesetzt und aufgearbeitet.
Ausbeute 1,1 g
Fp. 124 -25 °C
NMR ($d_6$-DMSO)δ = 3,75 ppm, s, 2H, $CH_2$-COOH
3,14 ppm, q, 2H, S-$CH_2$-$CH_3$
2,23 ppm, s, 3H, 4$CH_3$
1,33 ppm, tr, 3H, S-$CH_2$-$CH_3$

Beispiel 3:

Stufe 1

2-Benzylmercapto-4-methyl-1,3-thiazol-5-yl-essigsäuremethylester

2,03 g (0,01 Mol) 2-Mercapto-4-methyl-1,3-thiazol-5-yl-essigsäuremethylester werden entsprechend Beispiel 1, Stufe 2, mit Kaliumcarbonat und Benzylchlorid in Aceton umgesetzt und aufgearbeitet.
Ausbeute 3,3 g Öl 100 % d. Th.
NMR ($d_6$-DMSO)δ = 7,27 ppm, d, 5H, aromat.
4,40 ppm, s, 2H, Benzyl $CH_2$
3,83 ppm, s, 2H, $CH_2$-$COOCH_3$
3,60 ppm, s, 3H, $COOCH_3$
2,26 ppm, s, 3H, 4-$CH_3$

Stufe 2

2-Benzylmercapto-4-methyl-1,3-thiazol-5-yl-essigsäure

3,03 g (0,01 Mol) Verbindung aus Stufe 1 werden gemäß Beispiel 1, Stufe 3, mit 2n-Natronlauge versetzt und entsprechend dem genannten Beispiel aufgearbeitet.
Ausbeute: 1,3 g
Fp. 129 - 130 °C
NMR ($d_6$-DMSO)δ = 7,36 ppm, d, 5H, Phenyl
4,43 ppm, s, 2H, Benzyl $CH_2$
3,75 ppm, s, 2H, $CH_2$-COOH
2,28 ppm, s, 3H, 4-$CH_3$

Beispiel 4:

2-n-Propylmercapto-4-methyl-1,3-thiazol-5-yl-essigsäuremethylester

Stufe 1

2,03 g (0,01 Mol) Verbindung aus Beispiel 1, Stufe 1, werden analog Bsp. 1, Stufe 2, mit Kaliumcarbonat und n-Propyljodid in Aceton umgesetzt und entsprechend aufgearbeitet.
Ausbeute 3,9 g Öl 100 % d. Th.
NMR ($d_6$-DMSO)δ = 3,83, s, 2H, $CH_2$-$COOCH_3$
3,62, s, 3H, $COOCH_3$
3,10, t, 2H, $CH_2$-$CH_2$ $CH_2$

2,38, s, 3H, 4-$CH_3$
1,67, q, 2H, $CH_2$-$CH_2$-$CH_3$
0,97, t, 3H, $CH_2$-$CH_2$-$CH_3$

Stufe 2

2-n-Propylmercapto-4-methyl-1,3-thiazol-5-yl-essigsäure

4,1 g (0,0168 Mol) Verbindung aus Stufe 1 werden analog Beispiel 1, Stufe 3, mit 2n Natronlauge versetzt und entsprechend dem Beispiel 1, Stufe 3, aufgearbeitet.
Ausbeute 2,0 g = 52 % d. Th.
Fp. 79 - 80 °C

NMR ($d_6$-DMSO)δ = 12,60, br s, 1H, COOH
3,73, s, 2H, $CH_2$-COOH
3,11, m, 2H, $CH_2$-$CH_2$-$CH_3$
2,20, s, 3H, 4$CH_3$
1,70, q, 2H, $CH_2$-$CH_2$-$CH_3$
0,97, t, 3H, $CH_2$-$CH_2$-$CH_3$

Beispiel 5:

(2-Carboxymethylthio-4-methyl-1,3-thiazol-5-yl)-essigsäure

7,6 g 2-Mercapto-4-methyl-1,3-thiazol-5-yl-essigsäure werden in
50 ml 2N Natronlauge gelöst und mit
6,9 g Bromessigsäure versetzt. Man erhitzt 3 h auf dem Dampfbad und hält währenddessen den pH durch Nachstellen mit 2N NaOH bei 8. Nach Abkühlen wird die Lösung filtriert und angesäuert. Man erhält 6,5 g vom Fp. 180 °C.

| Elementaranalyse ($C_8H_9NO_4S_2$; 297,3) | | | | | | | | |
|---|---|---|---|---|---|---|---|---|
| ber.<br>gef. | C | 38,8 %<br>38,6 % | H | 3,7 %<br>3,6 % | N | 5,7 %<br>5,6 % | S | 25,9 %<br>25,5 % |

$^1$H-NMR ($d_6$-DMSO):δ(ppm): 2,25 (s, $CH_3$-Thiazol, 3H),
3,70 (s, 5-$CH_2$-Thiazol, 2H),
3,93 (s, 2-$CH_2$-S-Thiazol, 2H)

Beispiel 6:

⟨2-(2-Carboxy-ethylthio)-4-methyl-1,3-thiazol-5-yl⟩-essigsäure

3,14 g 2-Mercapto-4-methyl-1,3-thiazol-5-yl-essigsäure werden in
40 ml N-Natriumhydroxid-Lösung gelöst und mit 2,16 g 3-Chlorpropionsäure versetzt. Nach Zusatz weiterer 10 ml N-NaOH-Lösung wird 5 h auf dem Dampfbad erhitzt. Die Lösung wird angesäuert und zur Kristallisationsvervollständigung abgekühlt. Filtration und zweimaliges Umkristallisieren aus Essigester ergaben 2,5 g vom Fp. 124 °C

| Elementaranalyse ($C_9H_{11}NO_4S_2$; 261,3) | | | | | | |
|---|---|---|---|---|---|---|
| ber.<br>gef. | C | 41,4 %<br>41,3 % | H | 4,2 %<br>4,2 % | N | 5,4 %<br>5,4 % |

Durch Umkristallisation aus Wasser kann das Hydrat erhalten werden. 1,0 g ergaben 0,9 g vom Fp. 93 °C

| Elementaranalyse ($C_9H_{11}NO_4S_2$; 261,3 x $H_2O$; 279,3) | | | | | | | | |
|---|---|---|---|---|---|---|---|---|
| ber.<br>gef. | C | 38,7 %<br>39,2 % | H | 4,7 %<br>4,8 % | N | 5,0 %<br>4,9 % | S | 22,9 %<br>23,1 % |

$^1$H-NMR ($d_6$-DMSO):$\delta$(ppm) = 2,17 (s, 4-$CH_3$-Thiazol, 3H),
2,61 und 3,21 (2xt, J = 7Hz, -$CH_2CH_2$-, 4H),
3,72 (s, 4-$CH_2$-Thiazol, 2H)

Beispiel 7:

⟨2-(3-Carboxy-prop-1-yl-thio)-4-methyl-1,3-thiazol-5-yl⟩-essigsäure

Stufe 1

⟨2-(3-Methoxycarbonyl-prop-1-yl-thio)-4-methyl-1,3-thiazol-5-yl⟩-essigsäure

9,27 g 2-Mercapto-4-methyl-1,3-thiazol-5-yl-essigsäure-4-methoxybenzylester werden in
100 ml trockenem DMF gelöst und mit
7,0 g wasserfreiem, gemahlenem Kaliumcarbonat versetzt. Zu dieser Suspension wird tropfenweise
4,5 g 4-Chlorbuttersäuremethylester gegeben. Man läßt 6 h bei Raumtemperatur rühren, filtriert und entfernt das Lösungsmittel. Das Rohprodukt wird durch Chromatographie an Kiesegel mit Essigester/Cyclohexan (1 : 1) gereinigt. Nach Entfernen des Lösungsmittels aus den Produktfraktionen wird in ca.
20 ml Methylenchlorid gelöst und mit
10 ml Trifluoressigsäure versetzt. Man läßt 30 Min. bei Raumtemperatur rühren, entfernt Lösungsmittel im Vakuum, nimmt den Rückstand in
100 ml 2n-Natriumbicarbonatlösung auf, die mit Essigester extrahiert wird. Die wäßrige Phase wird angesäuert und erneut mit insgesamt
300 ml Essigester extrahiert. Die organischen Phasen werden getrocknet mit $MgSO_4$ und vom Lösungsmittel befreit. Man erhält 3,5 g Stufe 1 mit Fp. 80 °C nach Umkristallisation aus Essigester/Diisopropylether.

$^1$H-NMR ($CDCl_3$):

s,$\delta$ = 3,66; 3H
m,$\delta$ =

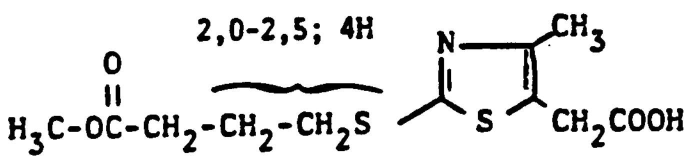


s,$\delta$ = 2,31; 3H
br. s;$\delta$ = 3,45; 1H
s,$\delta$ = 3,71; 2H
t,$\delta$ = 3,16; J = 7Hz; 2H

Stufe 2

⟨2-(3-Carboxy-prop-1-yl-thio)-4-methyl-1,3-thiazol-5-yl⟩-essigsäure

3,5 g der in Stufe 1 gewonnen Verbindung werden in
20 ml Methanol gelöst und mit
0,8 g NaOH und
10 ml Wasser 6 h bei Raumtemperatur gerührt. Man engt zur Trockene ein, nimmt erneut mit Wasser auf und säuert mit 2N HCl-Lösung an. Das so ausgefällte Produkt wird filtriert und aus Essigester umkristallisiert. Man erhält 1,8 g vom Fp.121 °C.

| Elementaranalyse ($C_{10}H_{13}NO_4S_2$; 275,3) | | | | | | | | |
|---|---|---|---|---|---|---|---|---|
| ber.<br>gef. | C | 43,6 %<br>43,8 % | H | 4,7 %<br>4,9 % | N | 5,1 %<br>5,0 % | S | 23,3 %<br>23,0 % |

$^1$H-NMR ($d_6$-DMSO):δ = 2,20 (s, 4-$CH_3$-Thiazol, 3H),
1,70-2,60 (m,2-$CH_2CH_2S$-Thiazol,4H),
3,15 (s, $HOOCCH_2$-, 2H),
3,73 (s, 5-$CH_2COOH$, 2H)

Die Beispiele 8 - 13 wurden wie in Beispiel 7 beschrieben synthetisiert:

Beispiel 8:

⟨2-(2-Carboxy-2-methoxy-imino-ethylthio)-4-methyl-1,3-thiazol-5-yl⟩-essigsäure

Fp. 138 °C

| Elementaranalyse ($C_{10}H_{12}N_2O_5S$, 304,3) | | | | | | | | |
|---|---|---|---|---|---|---|---|---|
| ber.<br>gef. | C | 39,5 %<br>39,4 % | H | 4,0 %<br>3,8 % | N | 9,2 %<br>9,3 % | S | 21,0 %<br>20,7 % |

$^1$H-NMR ($d_6$-DMSO):δ = 2,30 (s, 4-$CH_3$-Thiazol, 3H),
3,63 (s, $CH_2S$, 2H),
3,93 (s, N-$OCH_3$, 3H),
4,06 (s, 5-$CH_2$-Thiazol, 2H)

Beispiel 9:

⟨2-(2-Carboxy-2-hydroxy-imino-ethylthio)-4-methyl-1,3-thiazol-5-yl⟩-essigsäure

Fp. 166 -67 °C

| Elementaranalyse ($C_9H_{10}N_2O_5S$; 290,3) | | | | | | | | |
|---|---|---|---|---|---|---|---|---|
| ber.<br>gef. | C | 37,2 %<br>37,5 % | H | 3,5 %<br>3,6 % | N | 9,6 %<br>9,9 % | S | 22,1 %<br>22,4 % |

$^1$H-NMR ($d_6$-DMSO): δ = 2,23 (s, 4-$CH_3$-Thiazol, 3H),
3,79 (s, $CH_2S$, 2H),
4,03 (s, 5-$CH_2$-Thiazol, 2H)

Beispiel 10:

⟨2-(4-Carboxy-butylthio)-4-methyl-1,3-thiazol-5-yl⟩-essigsäure

Fp. 110 -11 °C

| Elementaranalyse ($C_{11}H_{15}NO_4S_2$; 289,3) | | | | | | |
|---|---|---|---|---|---|---|
| ber.<br>gef. | C | 45,7 %<br>45 % | H | 5,2 %<br>5,4 % | N | 4,8 %<br>4,7 % |

$^1$H-NMR ($d_6$-DMSO):δ = 1,6 und 2,2 und 3,1 (je m, -$(CH_2)_4$-8H),2,23 (s, 4-Thiazol-$CH_3$, 3H), 3,72 (s, 5-$CH_2$-Thiazol, 2H)

Beispiel 11:

⟨2-(5-Carboxy-pentyl-thio)-4-methyl-1,3-thiazol-5-yl⟩-essigsäure

Fp. 128 -29 °C

| Elementaranalyse ($C_{12}H_{17}NO_4S_2$; 303,3) | | | | | | |
|---|---|---|---|---|---|---|
| ber.<br>gef. | C | 47,5 %<br>47,6 % | H | 5,6 %<br>5,7 % | N | 4,6 %<br>4,5 % |

$^1$H-NMR ($d_6$-DMSO):δ = 1,5 und 2,2 und 3,2 (je m, $-(CH_2)_5$-10H),2,20 (s, 4-$CH_3$-Thiazol, 3H), 3,76 (s, 5-$CH_2$-Thiazol, 2H)

Beispiel 12:

⟨2-(1-Carboxy-1-methyl-ethylthio)-4-methyl-1,3-thiazol-5-yl⟩-essigsäure

Fp. 154 -55 °C

$^1$H-NMR ($d_6$-DMSO):δ = 1,48 (s, $(CH_3)_2C$, 6H)
2,25 (s, 4-$CH_3$-Thiazol, 3H),
3,80 (s, 5-$CH_2COOH$-Thiazol, 2H)

Beispiel 13:

⟨2-(1-Carboxy-1-cyclobutylthio)-4-methyl-1,3-thiazol-5-yl⟩-essigsäure

Fp. 114 -15 °C

$^1$H-NMR ($d_6$-DMSO):δ = 1,8 - 2,6 (m, 6H, Cyclobutyl-H),
2,20 (s, 4-$CH_3$-Thiazol, 3H),
3,75 (s, 5-$CH_2CO$)

Beispiel 14:

4-Carboxy-5-ethylthio-1,3-thiazol

6 g 5-Ethylthio-1,3-thiazol-4-carbonsäuremethylester wurden in 100 ml Methanol gelöst und mit 1,2 g NaOH in 10 ml Wasser ca. 1h unter Rückfluß erhitzt. Nach Entfernen des Lösungsmittels am Rotationsverdampfer, filtrierte man ab und säuerte mit 2N HCl-Lösung an. Nach dem Trocknen erhielt man 4,7 g der Titelverbindung vom Fp. 158 -59 °C.

$^1$H-NMR ($d_6$-DMSO):δ(ppm): 1,2 (t, Ethylthio-$CH_3$, J = 7Hz, 3H), 2,9 (q, Ethylthio-$CH_2$, J = 7Hz, 2H), 8,8 (s, Thiazol-2H, 1H).

MS: Mol peak 189 ($C_6H_7NO_2S_2$)

Beispiel 15:

5-Carboxymethylthio-1,3-thiazol-4-yl-carbonsäure

12,3 g 5-Methoxycarbonylmethylthio-1,3-thiazol-4-yl-carbonsäuremethylester wurden in 100 ml Methanol gelöst und mit 2 g NaOH in 20 ml Wasser 2h unter Rückfluß erhitzt. Nach Entfernen des Methanols am Rotationsverdampfer, klärte man durch Filtration und säuerte mit 2N HCl-Lösung an. Nach dem Trocknen erhielt man 10,3 g vom Fp. 237 °C.

$^1$H-NMR ($d_6$-DMSO):δ(ppm): 3,9 (s, $HOOCCH_2S$, 2H), 8,8 (s, Thiazol-2H, 1H),

IR (KBr-Preßling):ν = 1680 $cm^{-1}$ (Thiazol-COOH),
1710 $cm^{-1}$ (-$SCH_2COOH$).

Beispiel 16

5-Carboxymethylthio-1,3-thiazol-4-yl-carbonsäuredi-Natriumsalz

24 g der Dicarbonsäure aus Beispiel 32 werden mit 8,8 g NaOH und ca. 50 ml Wasser warm gelöst. Man versetzt bis zur schwachen Trübung mit Methanol und läßt unter Kühlung auskristallisieren. Filtration ergab 23,5 g Di-natriumsalz vom Fp. >300 °C.

| Elementaranalyse ($C_6H_3NO_4S_2Na_2$ x $H_2O$; 281,4) | | | | |
|---|---|---|---|---|
| ber.<br>gef. | $H_2O$ | 6,4 %<br>7,4 % | Na | 16,3 %<br>16,0 % |

Beispiel 17:

⟨4-(4-Carboxy-1,3-thiazol-5-yl)-thio⟩-buttersäure

4 g des Dimethylesters der Titelverbindung wurden in ca. 20 ml Ethanol gelöst und mit 1,5 g NaOH in 5 ml Wasser 1h unter Rückfluß erhitzt. Nach Entfernen des Lösungsmittels am Rotationsverdampfer und Filtration der wässrigen Lösung, säuert man mit 2N HCl-Lösung an. Nach Trocknen des abfiltrierten Niederschlages erhielt man 2,7 g Titelverbindung vom Fp. 177 °C.

$^1$H-NMR ($d_6$-DMSO):$\delta$(ppm): 1,9 (m, S-$CH_2CH_2CH_2COOH$, 2H), 2,4 und 3,1 (je t, S-$\underline{CH_2}CH_2\underline{CH_2}$ COOH, je 2H, J = 7Hz), 8,9 (s, Thiazol-2H).

IR (KBr-Preßling):$\nu$ = 1680 $cm^{-1}$ (Thiazol-4-COOH), 1707 $cm^{-1}$ (S-$(CH_2)_3$COOH).

Beispiel 18:

⟨3-(4-Carboxy-1,3-thiazol-5-yl)-thio⟩-propionsäure

13 g des Dimethylesters der Titelverbindung wurden in 200 ml Methanol gelöst und mit 4 g NaOH in 25 ml Wasser 2h auf dem Dampfbad unter Rückfluß erhitzt. Nach Abdestillieren des Methanols und Filtration der wässrigen Phase säuerte man mit 2N HCl-Lösung zur Ausfällung der Säure an. Man erhielt nach Trocknen 2,3 g vom Fp. 172 °C.

IR (KBr-Preßling):$\nu$ = 3090 $cm^{-1}$ (C-H-Thiazol), 1700 $cm^{-1}$ (COOH).

$^1$H-NMR ($d_6$-DMSO):$\delta$ (ppm): 2,7 und 3,2 (je t, -S-$CH_2CH_2$ COOH, J ≃ 7Hz, je 2H), 8,9 (s, Thiazol-2-H, 1H).

Beispiel 19:

⟨5-(4-Carboxy-1,3-thiazol-5-yl)-thio⟩-pentansäure Dinatriumsalz

20 g des Dimethylesters der Titelverbindungen wurden in 100 ml Methanol und 100 ml Wasser gelöst und mit 5,6 g NaOH versetzt. Man erhitzte ca. 1h unter Rückfluß, destillierte Methanol ab, filtrierte die Wasserphase und säuerte mit 2N HCl-Lösung an. Nach dem Trocknen erhielt man 15 g vom Fp. 187-190 °C. Diese Menge wurde in wenig Wasser mit 4,2 g NaOH gelöst und mit Aceton versetzt. Die entstandene Ausfällung wurde abfiltriert, getrocknet. Man erhielt 17,5 g Titelverbindung vom Fp. 210 °C (Zers.).

$^1$H-NMR ($D_2O$):$\delta$ (ppm): 1,7 und 2,2 und 3,0 (je m, -$SCH_2$ $CH_2CH_2CH_2CO_2$, 4 und 2 und 2H), 8,6 (s, Thiazol-2H, 1H).

| Elementaranalyse ($C_9H_9NO_4S_2Na_2$ x 1,5 $H_2O$; 332,3) | | | | | | | | | |
|---|---|---|---|---|---|---|---|---|---|
| ber.<br>gef. | C | 32,4%<br>32,5% | H | 3,6%<br>3,7% | N | 4,2%<br>4,1% | Na | 13,8%<br>14,0% | $H_2O$ | 8,1%<br>8,8% |

Beispiel 20:

⟨6-(4-Carboxy-1,3-thiazol-5-yl)-thio⟩-hexansäure Dinatriumsalz

20 g des Dimethylesters der Titelverbindung wurden in 200 ml Wasser/Methanol (1:1) mit 5,4 g NaOH 1h unter Rückfluß auf dem Dampfbad erhitzt. Nach Abdestillieren des Methanols; wurde die Wasserphase geklärt,mit 2N HCl-Lösung angesäuert und die Dicarbonsäure abfiltriert. Ausbeute 16,6 g vom Fp. 151 -52 °C. Die so erhaltene Menge wurde im 4,4 g NaOH in wenig Wasser gelöst und mit Aceton das Dinatriumsalz ausgefällt.
Ausbeute: 18,5 g vom Fp. 164 -66 °C (Zers.)

$^1$H-NMR ($D_2O$):δ(ppm): 1,6 und 2,2 und 3,0 (je m, -S-$(CH_2)_5$-$CO_2$, 6 und 2 und 2H), 8,6 (s, Thiazol-2H, 1H).

| Elementaranalyse ($C_{10}H_{11}NO_4S_2Na_2$ x $1H_2O$; 337,3) | | | | | | | | | | |
|---|---|---|---|---|---|---|---|---|---|---|
| ber.<br>gef. | C | 35,6%<br>34,8% | H | 3,9%<br>4,0% | N | 4,1%<br>4,0% | Na | 13,6%<br>13,3% | $H_2O$ | 5,4%<br>6,9% |

Beispiel 21:

(2-Carboxymethylthio-1,3-thiazol-5-yl)-essigsäure

2,97 g (2-Mercapto-1,3-thiazol-5-yl)-essigsäure wurden in 17 ml 2H NaOH-Lösung gelöst. Dazu gab man 2,78 g Bromessigsäure und erhitzte 3h auf dem Dampfbad unter Rückfluß. Nach dem Abkühlen wurde geklärt und mit 2H HCl-Lösung angesäuert. Die Auskristallisation über Nacht im Kühlschrank ergab 1,4 g vom Fp. 120-21 °C.

$^1$H-NMR ($d_6$-DMSO):δ(ppm): 3,65 (s, S-$CH_2$COOH, 2H)
3,8 (s, Thiazol-$CH_2$COOH, 2H),
7,3 (s, Thiazol-4H, 1H).

MS: $M^+$ = 233 ($C_7H_7NO_4S_2$)

Beispiel 22:

⟨2-(3-Carboxy-propylthio)-1,3-thiazol-5-yl⟩-essigsäure

1,5 g ⟨2-(3-Methoxycarbonylpropylthio)-1,3-thiazol-5-yl⟩-essigsäure wurden in 5 ml Methanol mit 7 ml wässriger 2H NaOH-Lösung 3h auf dem Dampfbad unter Rückfluß erhitzt. Nach Entfernen des Methanols am Rotationsverdampfer und Klärung der wässrigen Lösung säuerte man mit 2N HCl-Lösung an und erhielt nach Auskristallisation 0,6 g vom Fp. 97 °C.

$^1$H-NMR ($d_6$-DMSO):δ(ppm): 2,0 (m, S$CH_2CH_2CH_2$COOH, 2H), 2,4 und 3,2 (je t, S-$CH_2CH_2CH_2$COOH, je 2H, J = 7Hz), 3,8 (s, Thiazol $CH_2$COOH, 2H), 7,3 (s, Thiazol-4H, 1H).

IR (KBr-Preßling):ν = 3100 $cm^{-1}$ (Thiazol-4H-Valenz), 1680 $cm^{-1}$ und 1710 $cm^{-1}$ (COOH).

Beispiel 23:

⟨2-(2-Carboxybenzyl)-thio-4-methyl-1,3-thiazol-5-yl⟩-essigsäure

Stufe 1

⟨2-(2-Methoxycarbonylbenzyl)-thio-4-methyl-1,3-thiazol-5-yl⟩essigsäuremethylester

4,1 g 2-Mercapto-4-methyl-1,3-thiazol-5-yl-essigsäuremethylester wurden in 50 ml DMF gelöst und in Gegenwart von 5 g gemahlenem Kaliumcarbonat tropfenweise mit 5,1 g 2-Brommethyl-benzoesäuremethylester versetzt. Nach Abklingen der Wärmetönung rührte man 1h nach, filtrierte ab und zog DMF am Rotationsverdampfer ab. Der Rückstand wurde in Essigester aufgenommen und 2x mit Wasser gewaschen. Die organische Phase trocknete man und entfernte das Lösemittel i.Vak.. Man erhielt 5,6 g eines Öls.

$^1$H-NMR ($CDCl_3$):δ(ppm): 2,3 (s, Thiazol-4-$CH_3$, 3H), 3,60 (s, Thiazol-5-$CH_2$, 2H), 3,65 (s, Thiazol-5-$CH_2COOCH_3$, 3H), 3,8 (s, aromat. $COOCH_3$, 3H), 4,7 (s, aromat. $CH_2$,2H),

7,1 (m, aromat. H, 4H)

Stufe 2

Titelverbindung

3,8 g Stufe 1 werden in 35 ml Methanol gelöst und mit 0,9 g NaOH in 5 ml Wasser auf dem Dampfbad 2h unter Rückfluß erhitzt. Man entfernt Methanol am Rotationsverdampfer, klärt die wässrige Lösung und säuert mit 2N HCl-Lösung an. Das ausgefällte Produkt wird aus Isopropanol umkristallisiert. Ausbeute 1,5 g vom Fp. 199 °C.

$^1$H-NMR ($d_6$-DMSO):δ(ppm): 2,2 (s, Thiazol-4-$CH_3$, 3H), 3,6 (s, Thiazol-5-$CH_2$, 2H), 4,6 (s, $CH_2S$, 2H), 7,2-7,8 (m, aromat. H, 4H).

IR (KBr-Preßling):ν = 1680 $cm^{-1}$ und 1710 $cm^{-1}$ (COOH)

Die folgenden Beispiele wurden analog Beispiel 23 durchgeführt.

Beispiel 24

⟨2-(3-Carboxybenzyl)-thio-4-methyl-1,3-thiazol-5-yl⟩-essigsäure

Stufe 1

⟨2-(3-Methoxycarbonylbenzyl)-thio-4-methyl-1,3-thiazol-5-yl⟩-essigsäuremethylester

Aus
4,1 g 2-Mercapto-4-methyl-1,3-thiazol-5-yl-essigsäuremethylester und
5,1 g 3-Brommethylbenzoesäuremethylester erhielt man 5,0 g Öl.

$^1$H-NMR ($CDCl_3$):δ(ppm): 2,3 (s, Thiazol-4-$CH_3$, 3H), 3,60 und 3,62 (2 x s, Thiazol-5-$CH_2$ , 2H; Thiazol-5-$CH_2COOCH_3$, 3H), 3,8 (s, aromat. $COOCH_3$, 3H), 4,4 (s, aromat. $CH_2$, 2H), 6,8 - 7,8 (m, aromat. H, 4H).

Stufe 2

Titelverbindung

Aus
2,4 g Stufe 1 erhielt man 1,2 g Titelverbindung vom
Fp. 221 °C.

$^1$H-NMR ($d_6$-DMSO):δ(ppm): 2,25 (s, Thiazol-4-$CH_3$, 3H), 3,72 (s, Thiazol-5-$CH_2$, 2H), 4,5 (s, aromat. $CH_2$, 2H), 7,3 - 8,0 (m, aromat. H, 4H).

IR (KBr-Preßling):ν = 1710 $cm^{-1}$ (COOH), 2500 $cm^{-1}$ (OH)

Beispiel 25:

⟨2-(4-Carboxybenzyl)-thio-4-methyl-1,3-thiazol-5-yl⟩-essigsäure

Stufe 1

⟨2-(4-Methoxycarbonylbenzyl)-thio-4-methyl-1,3-thiazol-5-yl⟩-essigsäuremethylester

Aus
6,1 g 2-Mercapto-4-methyl-1,3-thiazol-5-yl-essigsäuremethylester und
8,6 g 4-Brommethyl-benzoesäuremethylester erhielt man 6,7 g Öl.

$^1$H-NMR ($CDCl_3$) = δ (ppm): 2,3 (s, Thiazol-4-$CH_3$, 3H), 3,55 (s, Thiazol-5-$CH_2$, 2H), 3,6 (s, Thiazol-5-$CH_2COOCH_3$, 3H), 3,78 (s, aromat. $COOCH_3$, 3H), 4,3 (s, aromat. $CH_2$, 2H), 7,25 und 7,80 (je d, J = 8Hz, aromat. H, 4H).

IR (Film):ν = 1710 $cm^{-1}$ und 1725 $cm^{-1}$ ($COOCH_3$)

Stufe 2

Titelverbindung

Aus
6,7 g Stufe 1 erhielt man 4,2 g der Titelverbindung vom Fp 196 °C (i-Propanol)

$^1$H-NMR ($d_6$-DMSO):δ (ppm) = 2,17 (s, 4-Thiazol-$CH_3$), 3H), 3,70 (s, 5-Thiazol-$CH_2$, 2H), 4,48 (s, aromat. $CH_2$, 2H), 7,35 und 7,85 (je d, J = 8Hz, aromat. H, 4H).

IR (KBr-Preßling):ν = 1700 $cm^{-1}$ (COOH), 2500 $cm^{-1}$ (OH)

| Elementaranalyse: $C_{14}H_{13}NO_4S$ (323,3) | | | | | | |
|---|---|---|---|---|---|---|
| ber.<br>gef. | C | 52,0 %<br>52,1 % | H | 4,1 %<br>4,2 % | N | 4,3 %<br>4,0 % |

Die Beispiele 26 und 27 wurden wie in Beispiel 5 beschrieben synthetisiert

Beispiel 26:

4-Carboxyethyl-2-carboxymethylthio-1,3-thiazol

NMR (DMSO - $d_6$) δ = 7,28 ppm (s, Thiazol-H)
δ = 4,1 ppm (s, $CH_2$) .
δ = 3,3 ppm (m, 2 x $CH_2$)

Beispiel 27:

4-Carboxyethyl-2-carboxyethylthio-1,3-thiazol

NMR (DMSO - $d_6$) δ = 7,18 ppm (S, Thiazol-H)
δ = 3,5 ppm (t, $CH_2$)
δ = 3,3 ppm (m, 2 x $CH_2$)
δ = 2,7 ppm (t, $CH_2$)

Beispiel 28:

4-Carboxy-2-carboxyethylthio-1,3-thiazol

6,9 g Brompropionsäure wurden in 100 ml Wasser gelöst und mit 1N NaOH auf pH 7 eingestellt. Diese Lösung wurde zu einer Suspension aus 7,5 g 4-Ethoxycarbonyl-2-mercapto-1,3-thiazol in 75 ml $H_2O$ getropft. Es wurde mit 2N NaOH auf pH 8-10 eingestellt, 4 h bei Raumtemperatur und 2 h bei 60 °C gerührt. Der pH wurde zwischen 8 und 10 gehalten. Unter Eiskühlung wurde mit 2N HCl auf pH 1 angesäuert und abfiltriert.
Ausbeute: 7,6 g der Titelverbindung

NMR (DMSO - $d_6$) δ = 8,35 ppm (s, Thiazol-H)
δ = 3,52 ppm (t, $CH_2$)
δ = 2,75 ppm (t, $CH_2$)

Beispiel 29:

5-Carboxy-2-carboxymethylthio-4-methyl-1,3-thiazol

Stufe 1:

6 g Bromessigsäure wurden in 50 ml Wasser gelöst und mit 1N NaOH auf pH 7-8 gestellt. Zu der auf 60 °C erhitzten Lsg. wurden 8 g 5-Ethoxycarbonyl-2-mercapto-4-methyl-1,3-thiazol gegeben und 2 h bei dieser Temperatur und bei pH 8 - 10 gerührt. Unter Eiskühlung wurde mit 2N HCl auf pH 1 angesäuert und

abgesaugt. 10,9 g

Stufe 2:

2,61 g des in Stufe 1 erhaltenen Produktes wurden in 20 ml Ethanol gelöst und mit 1,96 g KOH in 70 ml Ethanol versetzt. Es wurde ½ h bei 50 - 60° C gerührt, mit 60 ml $H_2O$ verdünnt und unter Eiskühlung mit ½ konz. HCl auf pH 0,5 angesäuert. Ethanol wurde am Rotationsverdampfer abgezogen, wobei das Produkt ausfiel. Es wurde filtriert und mit $H_2O$ gewaschen;
Ausbeute: 2,05 g der Titelverbindung

NMR (DMSO - $d_6$) δ = 4,08 ppm (s, $CH_2$)
δ = 2,52 ppm (s, $CH_3$)

Die Beispiele 30 - 31 wurden wie in Beispiel 29 beschrieben hergestellt.

Beispiel 30:

4-Carboxy-2-carboxymethylthio-1,3-thiazol

NMR (DMSO - $d_6$) δ = 8,23 ppm (s, Thiazol-H)
δ = 4,12 ppm (s, $CH_2$)

Beispiel 31:

5-Carboxy-2-carboxyethylthio-4-methyl-1,3-thiazol

NMR (DMSO - $d_6$) δ = 3,5 ppm (t, $CH_2$)
δ = 2,75 ppm (t, $CH_2$)
δ = 2,57 ppm (s, $CH_3$)

Beispiel 32:

4-Carboxy-2-carboxypropylthio-1,3-thiazol

Stufe 1:

8,3 g 4-Ethoxycarbonyl-2-mercapto-1,3-thiazol wurden in 100 ml Aceton zusammen mit 6,1 g $K_2CO_3$ und 10,3 g Brombuttersäureethylester ¾ h bei Raumtemperatur gerührt. Es wurde filtriert und das Filtrat zur Trockne eingedampft. Der Rückstand wurde in Essigester aufgenommen und 2 x mit $H_2O$ extrahiert. Nach Verdampfen des Lösungsmittels verblieben 14 g eines Öls.

Stufe 2:

13.8 g des Produktes aus Stufe 1 wurden in 80 ml Ethanol gelöst und mit 11,6 g KOH in 250 ml Ethanol versetzt. Es wurde 3 h bei Raumtemperatur gerührt, und das ausgefellene Kaliumsalz abfiltriert. Das Salz wurde in 150 ml Wasser gelöst und bei 0° C mit ½ konz. HCl auf pH 0,5 gestellt, wobei das Produkt ausfiel: 8,7 g der Titelverbindung

NMR (DMSO - $d_6$) δ = 8,22 (s, Thiazol-H)
ε = 3,25 (t, $CH_2$)
δ = 2,38 (t, $CH_2$)
δ = 1,92 (q, $CH_2$)

Die Beispiele 33 - 34 wurden wie in Beispiel 32 beschrieben hergestellt.

Beispiel 33:

5-Carboxy-2-carboxypropylthio-4-methyl-1,3-thiazol

NMR (DMSO - $d_6$) δ = 3,27 ppm (t, $CH_2$)
δ = 2,58 ppm (s, $CH_3$)
δ = 2,38 ppm (t, $CH_2$)

δ = 1,93 ppm (q, $CH_2$)

Beispiel 34:

5-Carboxy-2-carboxybutylthio-4-methyl-1,3-thiazol

NMR (DMSO - $d_6$) δ = 3,23 ppm (t, $CH_2$)
δ = 2,57 ppm (s, $CH_3$)
δ = 2,27 ppm (t, $CH_2$)
δ = 1,73 ppm (m, 2 x $CH_2$)

Beispiel 35:

5-Acetamido-2-carboxymethythio-1,3-thiazol

In 90 ml trockenem DMF löste man 3,48 g (20 mmol) 5-Acetamido-2-mercapto-1,3-thiazol,setzte 2,78 g (20 mmol) Bromessigsäure zu und rührte 2 Stunden bei Raumtemperatur. Dann zog man das Lösungsmittel im Vakuum ab und wusch den festen Rückstand mit Diethylether. Nach Umkristallisation aus Isopropanol/Diethylether erhielt man 3,1 g (67 %) der Titelverbindung.
$^1H$ - NMR ($d_6$ - DMSO): δ = 2,10 ppm (s, 3H, $-CH_3$), 3,93 ppm (s, 2H, $-CH_2-$), 7,40 ppm (s, 1H, Thiazol-H), 7,90 ppm (br s, $-CO_2H$ und $H_2O$), 11,43 ppm (br, s, 1H, -NH).

Analog wie in Beispiel 35 wurden die in den Beispielen 36 und 37 beschriebenen Substanzen synthetisiert.

Beispiel 36:

N-(2-Carboxymethylthio-1,3-thiazol-5-yl)-glutarsäuremonoamid

$^1H$ - NMR ($d_6$ - DMSO): ε = 1,56 - 2,65 ppm (m, 6H, $-CH_2-CH_2-CH_2-$), 3,90 ppm (s, 2H, $-CH_2S$), 7,36 ppm (s, 1H, Thiazol-H), 8,26 ppm (br s, $-CO_2H$ und $H_2O$), 11,40 ppm (br s, 1H, -NH-)

Beispiel 37:

N-(Carboxymethylthio-1,3-thiazol-5-yl)-bernsteinsäuremonoamid

$^1H$ - NMR ($d_6$ - DMSO): δ = 2,40 - 2,45 ppm (m, 4H, $-CH_2-CH_2-$), 3,90 ppm (s, 2H, $-CH_2S-$), 7,33 ppm (s, 1H, Thiazol-H), 9,26 ppm (br s, $-CO_2H$ und $H_2O$), 11,46 ppm (br s, 1H, -NH-)

Beispiel 38:

3-[2-(Carboxymethylthio)-4-methyl-1,3-thiazol-5-yl]-propionsäure

In 40 ml trockenem DMF legte man 2 g (8,6 mmol) (2-Mercapto-4-methyl-1,3-thiazol-5-yl)-3-propionsäureethylester vor und gab 1,49 g (8,6 mmol) Bromessigsäure hinzu. Nach 2 Stunden zog man das Lösungsmittel im Vakuum ab und erhielt 3,8 g 3-[2-(Carboxymethylthio)-4-methyl-1,3-thiazol-5-yl]-propionsäureethyl-ester als Öl. Das Rohprodukt nahm man in 25 ml Ethanol auf, setzte 33 ml 1 n Natronlauge zu und erwärmte das Reaktionsgemisch auf 50° C. Nach beendeter Reaktion zog man das Ethanol im Vakuum ob, nahm den wäßrigen Rückstand mit konz. Salzsäure bei pH 3,0 auf und extrahierte mit Essigester. Aus der organischen Phase erhielt man nach dem Trocknen mit $MgSO_4$, Einengen und Kristallisieren mit Diethylether 1,6 g (71 %) der Titelverbindung.
$^1H$ - NMR ($d_6$ - DMSO): δ = 2,23 ppm (s, 3H, $-CH_3$), 2,25 - 3,15 ppm (m, 4H, $-CH_2-CH_2-$), 3,96 ppm (s, 2H, $-CH_2S-$).

Beispiel 39:

3-[2-(3-Carboxyprop-1-ylthio)-4-methyl-1,3-thiazol-5-yl]-propionsäure

In 40 ml trockenem DMF legte man 2 g (8,6 mmol) (2-Mercapto-4-methyl-1,3-thiazol-5-yl)3-propionsäureethylester vor, gab 1,48 g (11 mmol) gepulvertes $K_2CO_3$ und 1,23 ml (8,6 mmol) 4-Brombuttersäureethylester zu und rührte das Reaktionsgemisch 2 Stunden bei 50 °C. Der festen Rückstand saugte man ob, zog das DMF im Vakuum ab und erhielt 3,6 g eines Rohproduktes, das man in 25 ml Ethanol aufnahm. Nach Zugabe von 1 n Natronlauge rührte man noch 2 Stunden bei Raumtemperatur, zog das Ethanol ab und stellte den wäßrigen Rückstand mit Salzsäure auf pH 3 ein. Man extrahierte mit Essigester, trocknete die organische Phase mit $MgSO_4$ und engte ein. Man erhielt 2,2 g (88 %) der Titelverbindung als Öl.

$^1H$ - NMR ($CDCl_3$) : δ = 1,66 - 2,5 und 2,6 - 3,15 ppm (2 m, 10H, 5 $CH_2$-Gruppen), 2,16 ppm (s, 3H, $-CH_3$), 6,4 ppm (br s, 2H, $-CO_2H$).

Beispiel 40.

5-(4-Chlorbenzylthio)-1,3-thiazol-4-yl-carbonsäure

13 g Methylester der Titelverbindung werden in 300 ml Methanol gelöst. Dazu gibt man 2 g NaOH gelöst in 10 ml Wasser und erhitzt 30 min auf dem Dampfbad. Beim Abkühlen kristallisiert das Na-Salz der Säure aus. Man entfernt das Lösungsmittel i. Vak., nimmt in Wasser auf und säuert mit 2 N HCl an. Nach Filtration und Trocknen erhält man 11 g Produkt mit Fp. 176 - 78 °C.

| Elementaranalyse ($C_{11}H_8NO_2S_2Cl$x0,7 $H_2O$; 298,3) | | | | | | | | |
|---|---|---|---|---|---|---|---|---|
| ber.<br>gef. | C | 44,3 %<br>44,6 % | H | 3,1 %<br>3,0 % | N | 4,7 %<br>4,7 % | $H_2O$ | 4,2 %<br>4,4 % |

$^1H$ - NMR ($d_6$ - DMSO):
δ (ppm) = 4,3 (s, $CH_2$, 2H)
= 7,3 (s, aromat. H, 4H)
= 8,9 (s, Thiazol-2-H, 1H)

Beispiel 41:

5-(2-Carbomethoxy-ethylthio)-1,3-thiazol-4-yl-carbonsäure

7,8 g des 5-(2-Carbomethoxy-ethylthio)-1,3-thiazol-4-yl-carbonsäure-tert.-butylesters werden 30 min bei Raumtemperatur in 100 ml Trifluoressigsäure, gemischt mit 100 ml Methylenchlorid, gerührt. Man entfernt das Lösungsmittel im Vakuum und rührt den Rückstand mit 100 ml Ether/Ligroin (50 - 70 °C) 1:1 aus. Nach Filtration und Trocknen erhält man 6,2 g vom Fp. 112 -16 °C, nach Umkristallisation aus Essigester Fp. 120 -21 °C.

| Elementaranalyse ($C_8H_9NO_4S_2$; 247,3) | | | | | | | | |
|---|---|---|---|---|---|---|---|---|
| ber.<br>gef. | C | 38,9 %<br>38,9 % | H | 3,7 %<br>3,8 % | N | 5,7 %<br>5,4 % | S | 25,9 %<br>25,8 % |

$^1H$ - NMR ($d_6$ - DMSO):
δ (ppm) = 2,7 und 3,2 (je t, $-S-CH_2CH_2-COO$, 4H)
= 3,6 (s, $COOCH_3$, 3H)
= 8,8 (s, Thiazol-2-H, 1H)

Beispiel 42:

2-(3-Carboxy-propylthio)-4-methyl-1,3-thiazol-5-yl-essigsäuremethylester

Stufe 1:

2-[3-(4-Methoxybenzyloxycarbonyl)-propylthio]-4-methyl-1,3-thiazol-5-yl-essigsäuremethylester

10,2 g 2-Mercapto-4-methyl-1,3-thiazol-5-yl-essigsäuremethylester werden in 200 ml Aceton gelöst und mit 12,1 g 4-Chlorbuttersäure-4-methoxybenzylester in Gegenwart von 13.0 g pulverisiertem trocknem Kaliumcarbonat (Pottasche) während 4 h intensiv gerührt unter Einhaltung der Umgebungstemperatur. Nach Filtration wird das Aceton im Vakuum entfernt und der Rückstand säulenchromatographisch gereinigt ($SiO_2$, Essigester-Cyclohexan 1 : 1).
Ausbeute: 21 g ölige Verbindung

$^1H$ - NMR ($CDCl_3$):
δ (ppm) = 2,0 (m, -S $CH_2CH_2CH_2COO$-, 2H)
= 2,2 (s, 4-$CH_3$-Thiazol, 3H)
= 2,3 und 3,2 (je t, -$SCH_2CH_2CH_2COO$-, 4H)
= 3,6 (s, $CH_2COOCH_3$, 2H)
= 3,65 und 3,7 (je s, aromat. $OCH_3$ und $COOCH_3$, je 3H)
= 4,9 (s, $CH_2$-Arom., 2H)
= 6,8 und 7,2 (je d, aromat. H, 4H)

Stufe 2:

Titelverbindung

5,3 g aus Stufe 1 werden mit 20 ml Trifluoressigsäure gelöst und nach 30 min Rühren im Vakuum eingeengt. Der Rückstand wird aus Essigester/Cyclohexan (2 : 1) 2 x umkristallisiert. Min erhält 1,6 g Produkt vom Fp. 104 -05° C.

| Elementaranalyse ($C_{11}H_{15}NO_4S_2$ ; 289,3) | | | | | | | | |
|---|---|---|---|---|---|---|---|---|
| ber.<br>gef. | C | 45,6 %<br>45,3 % | H | 5,2 %<br>5,5 % | N | 4,8 %<br>4,7 % | S | 22,2 %<br>22,0 % |

$^1H$ - NMR ($CDCl_3$)
δ (ppm) = 2,0 (m, -$SCH_2CH_2CH_2COO$-, 2H)
= 2,3 (s, 4-$CH_3$-Thiazol, 3H)
= 2,4 und 3,2 (je t, -$SCH_2CH_2CH_2COO$-, 4H)
= 3,6 (s, $COOCH_3$, 3H)
= 10,8 (s, COOH, 1H, austauschbar mit $D_2O$)

**Patentansprüche**

**1.** Sulfide der allgemeinen Formel II

Het'-S-$R^2$ (II)

in der Het' für 1,3-Thiazolyl steht, das ein- oder zweifach substituiert ist durch $C_1$-$C_6$-Alkyl; durch Carboxy-$C_1$-$C_6$-alkyl; durch Carboxy oder durch $C_2$-$C_5$-Acylamino, wobei Acyl für den Rest einer aliphatischen Dicarbonsäure steht,
wobei Het' mindestens eine direkt gebundene Carboxylgruppe oder einen der vorstehend aufgeführten, eine Carboxylgruppe enthaltenden Substituenten trägt und $R^2$ die Bedeutung besitzt von $C_1$-$C_6$-Alkyl, von $C_1$-$C_6$-Alkyl, das substituiert ist durch Carboxy, $C_1$-$C_4$-Alkoxycarbonyl, Hydroximino, $C_1$-$C_4$-Alkoximino oder Phenyl, das durch Carboxy oder Halogen substituiert sein kann oder von $C_3$-$C_6$-Cycloalkyl, das durch Carboxy substituiert sein kann,
wobei die vorstehend genannten Carboxylgruppen auch in Form ihrer physiologisch verträglichen Salze vorliegen können.

**2.** Sulfide der allgemeinen Formel II

Het'-S-$R^2$ (II)

worin Het' für

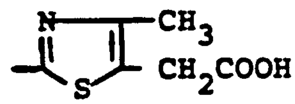

und $R^2$ für $HOOC-(CH_2)_n$-mit n = 1 - 5, steht.

**3.** Sulfide gemäß Anspruch 2, wobei n = 3 ist.

**4.** Verfahren zur Herstellung der Sulfide der allgemeinen Formel II, dadurch gekennzeichnet, daß man
a) eine Verbindung der allgemeinen Formel III

Het'-SH (III)

in der Het' die vorstehende Bedeutung hat, mit einem Alkylierungsmittel der allgemeinen Formel IV

$R^2$-X (IV)

in der $R^2$ die oben angegebene Bedeutung hat und X für eine leicht abspaltbare Gruppe steht, umsetzt oder
b) eine Verbindung der allgemeinen Formel V

Het'-X (V)

in der Het' und X die vorstehenden Bedeutungen haben, mit einer Verbindung der allgemeinen Formel VI

$R^2$-SH (VI)

in der $R^2$ in der oben angegebenen Weise definiert ist, umsetzt.

**5.** Pharmazeutische Mittel, gekennzeichnet durch einen Gehalt aneiner Verbindung der allgemeinen Formel II.

## Claims

**1.** A sulfide of the general formula

Het'-S-$R^2$ (II)

in which Het' is 1,3-thiazolyl which is mono- or disubstituted by $C_1$-$C_6$-alkyl; by carboxy-$C_1$-$C_6$-alkyl; by carboxyl or by $C_2$-$C_5$-acylamino, where acyl is the radical of an aliphatic dicarboxylic acid,
where Het' carries at least one directly bonded carboxyl group or one of the substituents containing a carboxyl group and listed above, and
$R^2$ means $C_1$-$C_6$-alkyl, $C_1$-$C_6$-alkyl which is substituted by carboxyl, $C_1$-$C_4$-alkoxycarbonyl, hydroximino, $C_1$-$C_4$-alkoximino or phenyl which can be substituted by carboxyl or halogen, or means $C_3$-$C_6$-cycloalkyl which can be substituted by carboxyl,
where the abovementioned carboxyl groups can also be in the form of their physiologically tolerated salts.

**2.** A sulfide of the general formula II

Het'-S-$R^2$ (II)

in which Het' is

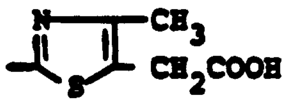

and $R^2$ is HOOC-$(CH_2)_n$- with n = 1-5.

**3.** A sulfide as claimed in claim 2, where n = 3.

**4.** A process for the preparation of a sulfide of the general formula II, which comprises
a) reaction of a compound of the general formula III

Het'-SH (III)

in which Het' has the abovementioned meaning, with an alkylating agent of the general formula IV

$R^2$-X (IV)

in which $R^2$ has the abovementioned meaning, and X is a group which can readily be eliminated, or
b) reaction of a compound of the general formula V

Het'-X (V)

in which Het' and X have the abovementioned meanings, with a compound of the general formula VI

$R^2$-SH (VI)

in which $R^2$ is defined in the abovementioned manner.

**5.** A pharmaceutical agent containing a compound of the general formula II.

**Revendications**

**1.** Sulfures de formule générale II

Het'-S-$R^2$ (II)

dans laquelle Het' représente un radical 1,3-thiazolyle qui est une ou deux fois substitué par un groupe alkyle en $C_1$-$C_6$;
par un groupe carboxyalkyle($C_1$-$C_6$); par un groupe carboxy ou par un groupe acylamino en $C_2$-$C_5$, le fragment acyle représentant le reste d'un acide dicarboxylique aliphatique;
Het' portant au moins un groupe carboxy fixé directement ou l'un des substituants comportant un groupe carboxy, indiqués précédemment; et
$R^2$ a la signification d'un radical alkyle en $C_1$-$C_6$, d'un radical alkyle en $C_1$-$C_6$ qui est substitué par un groupe carboxy, alcoxy($C_1$-$C_4$)-carbonyle, hydroximino, alcoxy($C_1$-$C_4$)-imino ou phényle qui peut être substitué par un atome d'halogène ou par un groupe carboxy, ou d'un radical cycloalkyle en $C_3$-$C_6$ qui peut être substitué par le groupe carboxy, les groupes carboxy précités pouvant également se trouver sous forme de leurs sels physiologiquement acceptables.

**2.** Sulfures de formule générale II

Het'-S-$R^2$ (II)

dans laquelle Het' représente

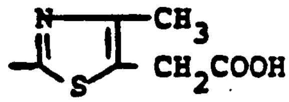

et $R^2$ représente $HOOC-(CH_2)_n-$ avec $n = 1-5$.

**3.** Sulfures selon la revendication 2, dans lesquels $n = 3$.

**4.** Procédé pour la préparation des sulfures de formule générale II, caractérisé en ce que
a) on fait réagir un composé de formule générale III

Het'-SH (III)

dans laquelle Het' a la signification donnée précédemment, avec un agent d'alkylation de formule générale IV

$R^2$-X (IV)

dans laquelle $R^2$ a la signification donnée précédemment et X représente un groupe aisément séparable, ou
b) on fait réagir un composé de formule générale V

Het'-X (V)

dans laquelle Het' et X ont les significations données précédemment, avec un composé de formule générale VI

$R^2$-SH (VI)

dans laquelle $R^2$ est tel que défini plus haut.

**5.** Compositions pharmaceutiques caractérisées par une teneur en un composé de formule générale II.